# EUROPEAN PATENT APPLICATION

(11) **EP 4 276 849 A1**
(43) Date of publication of application: **15.11.2023**
(21) Application number: 22198415.6
(22) Date of filing: 28.09.2022
(51) Int. Cl.: G16H 40/60, A61B 6/00

(54) **HYBRID MEDICAL IMAGING SYSTEM**

(71) Applicant: Siemens Healthcare GmbH, 91052 Erlangen (DE)
(72) Inventor: Polacin, Arkadiusz, 91080 Uttenreuth (DE); Simsekli, Fahri, 91301 Forchheim (DE); Brandstetter, Veronika, 81673 München (DE); Eckert, Florian, 90425 Nürnberg (DE); Hinrichs, Merten, 90489 Nürnberg (DE); Mühleck, Stefan, 91475 Lonnerstadt (DE); Schmidt, Matthias, 91083 Baiersdorf (DE); Seidel-Schober, Cathleen, 90556 Cadolzburg (DE)

(57) **Abstract**

Various examples of the disclosure pertain to a hybrid medical imaging system that includes hardware and software components. The hardware components implement physical functions, and the software components simulate other physical functions. Thereby, system testing can become possible at an early stage during development.

## Description

Various examples of the disclosure generally relate to hybrid medical imaging systems.

During early development phases of complex medical imaging systems including software (SW) and hardware (HW)components, for example development of computed tomography (CT) systems, not all HW components may be available at any point in time during the development cycle. Missing HW components may hinder developing and testing medical imaging systems in numerous ways.

Missing HW components may limit and slow down the system integration. The SW system needs a stable HW system for testing and the SW development teams often need to wait until a stable HW system is available. During integration of a HW component into the HW system, often effects from other HW components are experienced. It is difficult to exclude impacts originating from other HW components during component integration into system.

Further, extensive testing may be limited because of environmental side effects, e.g., of X-Rays. Some HW components, e.g., a HW component emitting X-rays, must be replaced after a while because of aging. Product validations in a test center may be able to validate only on a final medical imaging system with a stable HW and SW systems. Some components may only be fully tested when the components are integrated into the medical imaging system.

All this makes it difficult to develop and test medical imaging systems that include multiple components.

According to conventional solutions, developing a medical imaging system during pre-integration (e.g., integration of HW components that may be assembled into the final HW system during system integration) may be performed with HW components that already exist. Not-yet available components are replaced with alternative older components, which may not have all characteristics of the replaced final HW component.

Such techniques are time-consuming and may require significant efforts for adaptation of existing HW components to match the requirements of the tested medical imaging system, e.g., enable interoperability with other HW components and/or SW components.

A plurality of sequential imaging system prototypes may have to be built and made available to developers for their integrations. Therein, the existing imaging system prototypes may be expanded by newer HW components as soon as they arrive. If the prototype is not expandable, it only may be used for simple tasks or tests.

Oftentimes, a component supplier performs simple physical HW component tests using samples without the complete imaging system available and the tests have to be repeated on site of the complete medical imaging system after delivery of a sample physical HW component. Accordingly, many iterations of development cooperation may be required between the medical imaging system producer and the HW component supplier. For example, a test department may need an own complete medical imaging systems for testing their own test cases. This means that an integrated system is requirement including all components. For further desired features and add-ons, additional medical imaging systems must be built for system validation, e.g., CT imaging systems with different patient handling systems.

Therefore, the idea of the presented approach is to provide advanced techniques for developing and testing medical imaging systems, which overcome or mitigate at least some of the limitations and disadvantages mentioned.

This task is solved by the features of the independent claims. Further advantageous examples are included in the dependent claims.

In the following, the solution according to the invention is described with regard to the claimed methods as well as with regard to the claimed medical imaging systems, wherein features, advantages, or alternative embodiments can be assigned to the other claimed objects and vice versa. For example, the claims related to the methods may be improved with features described in the context of the medical imaging systems.

According to an example, a hybrid medical imaging system includes both a hardware component as well as a simulation software (SW) component. The hardware component is configured to provide an execution of a first physical function of the hybrid medical imaging system. The simulation SW (S-SW) component is configured to provide a simulated execution of a second physical function of the hybrid medical imaging system.

The S-SW component can in other words mimic an associated hardware (HW) component. It is thus dispensable to include the HW component in the hybrid medical imaging system.

The simulated execution of the second physical function may be dependent on the execution of the first physical function. In other words, the hardware component and the simulation software (S-SW) component can interact during the execution of the first physical function and the simulated execution of the second physical function. They may exchange control data. They may operate based on the same time base. They may be controlled by the same control component.

Thereby, global functionality of a medical imaging system can be simulated by using the hybrid medical imaging system. For instance, a control component can be configured to control, both, the HW component as well as the S-SW component in accordance with an imaging protocol. The imaging protocol may be defined by a set of control commands for SW components and HW components of the medical imaging system to jointly operate to facilitate acquisition of imaging data. Thus, the hybrid medical imaging device may be functional to acquire simulated imaging data.

For instance, control software of a medical device which is controlling a specific hardware component does not recognize any difference between the real hardware component and the simulation SW component which is simulating this component. As a result, in a hybrid medical imaging system, S-SW and HW components jointly operate to facilitate acquisition of imaging data.

According to a further example, a method includes a HW component in a hybrid medical imaging system. The HW component is configured to provide an execution of a first physical function of the hybrid medical imaging system. The method further comprises including a S-SW component in the hybrid medical imaging system. The S-SW component is configured to provide a simulated execution of a second physical function of the hybrid medical imaging system. The method further includes replacing the S-SW component by a further HW component in the hybrid medical imaging system. The further HW component is configured to provide an execution of the second physical function of the hybrid medical imaging system.

By the disclosed techniques, the following advantages may be realized.

It may not be necessary to wait until HW components are available to test functionality of a medical imaging system by means of the hybrid medical imaging system. HW components can be integrated at different points in time into a hybrid medical imaging system.

Where a HW component is not available, it may be replaced by a S-SW component.

The integration of different HW components may be executed separately. Interaction between different HW components can be considered by using respective S-SW components.

Suppliers of HW components may be provided with a hybrid medical imaging system and they can integrate their components by themselves, which leads to less efforts for system integration.

System pre-integration may be started on a hybrid medical imaging system before having a real system with complete physical HW components; in such a way system (pre-)integration may be shortened.

Many HW component variants may be supported and tested within one hybrid medical imaging system, e.g., a plurality of patient handling system variants or rotating drive variants of a computed tomography (CT) gantry.

For instance, when using an S-SW component that simulates an X-ray source of a CT medical imaging system, no lead protected test rooms may be necessary, because no physical X-rays are needed for simulation. A hybrid medical imaging system may be placed without any limitation in any room. For example, it may be placed in offices, labs, or small rooms. A real CT medical imaging system may need app. 30 m² area and a lead protected room. A hybrid medical imaging system on the other hand needs app. 1 m² area and no lead protection.

No aging of the simulated HW components occurs and it might not be necessary to replace them on the hybrid medical imaging system. For example, after some 100 or 1000 operating hours on real system X-Ray-component shall be replaced with a new one.

Extensive testing becomes possible, and reproducible system performance tests may be done on a hybrid medical imaging system.

The hybrid medical imaging system may be used remotely. This is not possible with a real CT system, e.g., because remote radiation enabling is prohibited by law. The hybrid medical imaging system may be available for people outside of the R&D area, e.g., sales manager or product manager.

A new hybrid medical imaging system may be made available to customers at the beginning of a project phase and enables to collect feedback from the customer. The feedbacks can be implemented very early in the project.

Customers may be able to execute their performance or function using tests on a hybrid medical imaging system before they buy the features or performance options.

It is to be understood that the features mentioned above and features yet to be explained below can be used not only in the respective combinations indicated, but also in other combinations or in isolation, without departing from the scope of the present disclosure. In particular features of the disclosed embodiments may be combined with each other in further embodiments.

The above summary is therefore only intended to give a brief overview of some features of some embodiments and implementations and is not to be understood as a limitation. Other embodiments may include features other than those described above.

The present disclosure is described in more detail below with reference to the accompanying drawings, in which like reference numerals refer to like elements.
FIG. 1A schematically illustrates a hybrid medical imaging system according to various examples.
FIG. 1B schematically illustrates a medical imaging system associated with the hybrid medical imaging system according to FIG. 1A according to various examples.
FIG. 2A schematically illustrates an exemplary CT imaging system 100, according to various examples.
FIG. 2B schematically illustrates an exemplary hybrid CT imaging system 100, according to various examples.
FIG. 3 schematically illustrates an exemplary computing device implementing a simulation SW component according to various examples.
FIG. 4 schematically illustrates steps of a method according to various examples.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in HW, firmware, software, or a combination thereof.

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

Various techniques described herein generally relate to hybrid medical imaging system, i.e., systems that use a combination HW and S-SW components. The HW components can provide an execution of physical functions. The S-SW components can provide a simulation of respective physical functions.

For instance, as a general rule, such simulated execution of a physical function can be implemented using, e.g., one or more of the following: an analytical model; a machine-learning algorithm; a numerical simulation such as a finite-element simulation; etc.

The HW and S-SW components can interact with each other to provide the execution and the simulated execution of physical functions, respectively. In other words, there a physical interaction can be simulated between the physical functions that are executed or simulated, respectively.

Examples of physical interactions are a DC electric field; an AC electric field; a DC magnetic field; an AC magnetic field; a vibration; a temperature gradient; a pressure gradient; X-ray radiation; electromagnetic interference; etc.

The simulated execution of a physical function can thus be dependent on the execution of another physical function by a HW component. Alternatively or additionally, the execution of a physical function by an HW component can be dependent on the simulated execution of another physical function by an S-SW component.

Thus, such hybrid medical imaging systems are partly using simulated HW components (or, more precisely, a simulation of a physical function of the simulated HW component is provided by respective S-SWs) and partly using real physical HW components. The hybrid medical imaging systems thus implement modular systems, wherein physical and simulated HW components may be modularly exchanged against each other.

S-SW component(s) may be implemented by computing devices. A simulation HW (e.g. simulator, or simulator card) may be used. The computing device or computing devices implementing one or more simulation software components and the hardware components can all be included in a common housing, e.g., a server rack. S-SW components can also, in some examples, be implemented at a cloud server.

According to examples, a communication interface may be provided for a simulation software component. The communication interface can be implemented in hardware, i.e., a communication interface hardware component can be provided. Such communication interface hardware component can be configured to communicate with the hardware component and/or the S-SW component. Specifically, a communication protocol used for a HW component that is mimicked by the S-SW component can be supported by the communication interface HW component. This oftentimes means that the communication interface HW component is configured to communicate with a HW component using a data bus communication protocol or a proprietary communication protocol and to communicate with the simulation software component using another communication protocol, e.g., a Transport Control Protocol/Internet Protocol (TCP/IP).

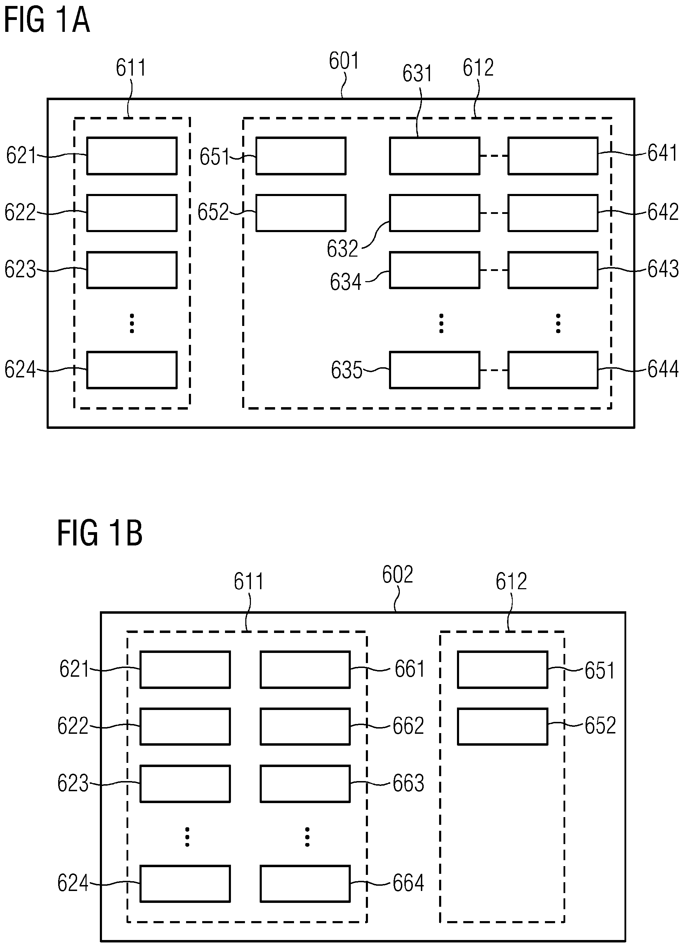

Fig. 1A schematically illustrates a hybrid medical imaging system 601. The hybrid medical imaging system 601 implements a digital twin of a medical imaging system that will be discussed later on in connection with FIG. 1B. Specifically, the digital twin partly relies on software, and partly relies on hardware.

The hybrid medical imaging system 601 includes a HW subsystem 611 and a software subsystem 612. The HW subsystem 611 includes multiple HW component 621-624 that are configured to provide execution of respective physical functions. The software subsystem 612 includes multiple S-SW components 641-644 that are configured to provide a simulated execution of respective physical functions. Also, the software component 612 includes computing component 631-635, e.g., a processor coupled to a memory, associated with the S-SW component 641-644 and configured to simulate the physical interactions between physical functions provided by the HW component 621-624 and the physical functions simulated by the S-SW components 641-644. Different physical functions can result in physical interactions. For instance, movement of a rotation of a gantry (i.e., a first physical function) can cause vibration (i.e., a physical interaction) that in turn affects operation of a patient handling system (i.e., a second physical function). Such physical interactions can also be tested through simulation.

As illustrated in FIG. 1A, the hybrid medical imaging system 601 also includes "native" software components 651, 652 that can implement, e.g., control functionality of the various components (a control component 651), user interaction by providing a human machine interface, etc. The SW components 651, 652 are thus configured to provide an execution of software functions of the hybrid medical imaging system.

Fig. 1B implements a medical imaging system 602. The medical imaging system 602 is associated with the digital twin of the hybrid medical imaging system 601 of FIG. 1A.

The medical imaging system 602 includes the HW components 621-624 that are also included in the hybrid medical imaging system 601. Also, the medical imaging system 602 includes the native software component 651, 652. The medical imaging system 602 also includes HW component 661-664 which are associated with the simulated action software component 641-644. In other words, the HW component 661-664 provide execution of the physical functions for which the S-SW components 641-644 provide simulated execution. Also, the computing component 631-635 are not required anymore, because the physical interactions are inherently present between the HW component 621-624 and 661-664.

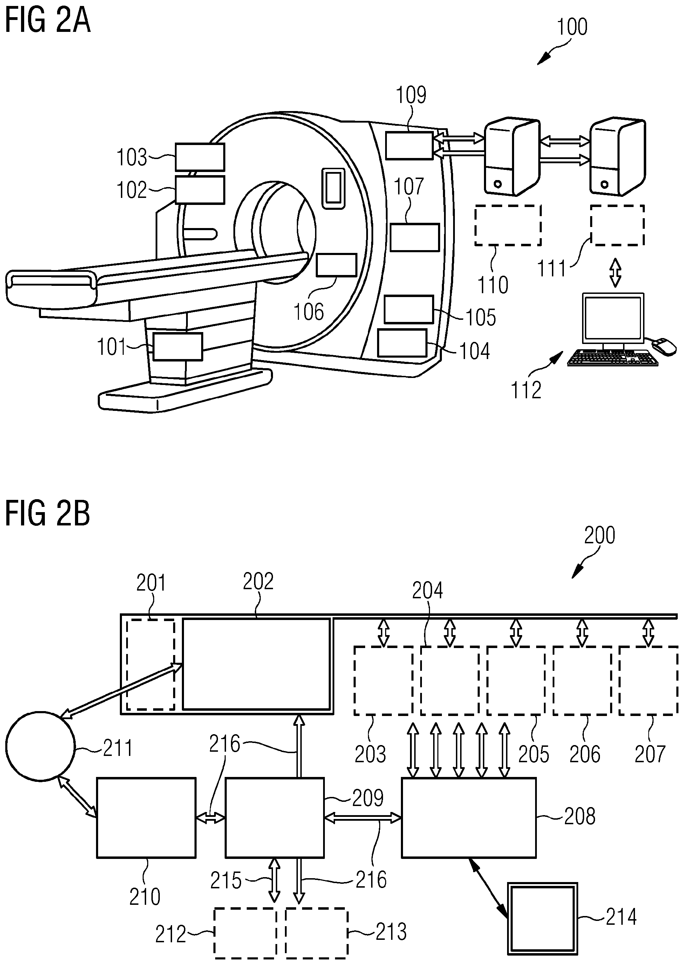

Next, an example implementation of such a medical imaging system that can be implemented by a digital twin is discussed in connection with FIG. 2A.

FIG. 2A schematically illustrates an overview of an exemplary CT medical imaging system 100, according to various examples.

As can be seen in FIG. 2A, the CT system 100 includes a HW subsystem with physical HW components(indicated by dashed lines) and SW subsystem with software components (indicated by solid lines).

In the example of FIG. 2A, the CT medical imaging system 100 includes for example a patient handling system 101 (e.g., a table) for moving a patient in and out of gantry 104, an X-Ray system 103 with a collimator 102, a detector 106, a rotation drive 105, and a plurality of other HW components 107.

The CT system includes a SW subsystem that includes for example a control component 109, an image reconstruction SW subsystem 110 and imaging control SW subsystem 111, which may be controlled by a user 112. These are native SW components. Control component can control the various HW components 101-107, e.g., based on an imaging protocol that intends to acquire imaging data.

Some of the HW components 101-109 may be replaced by respective S-SW components (not illustrated in FIG. 2A but corresponding to the scenario of FIG. 1A/1B). For instance, in the example of FIG. 2A, the physical components 101-107 marked by dashed lines as part of the HW system are determined and identified as replaceable with simulated HW components, in other words each of these physical HW components may be replaced by a respective simulator device.

The simulated HW components may be connected to and interfaced with the medical imaging system using the same, i.e. original, physical interfaces and may communicate with the medical imaging system using the same communication protocols as the respective original physical HW component. For this purpose, each S-SW component may be associated with a respective HW component that implements such communication interface. Thereby, HW components and S-SW can communicate with each other.

By using such communication HW components, the same communication speed between S-SW and HW components of a hybrid medical imaging system can be used as in-between respective HW components. Real-time communication is enabled.

Thus, S-SW components may be realized with the same communication interfaces as the original HW components.

Thus, from the perspective of a HW component, it may be transparent whether the HW component communicates with another HW component or with a respective S-SW component.

In various examples, for example UART, CAN, CANOpen, SiDaNet, or similar data bus communication protocols, communication network protocols (e.g., Transport Control Protocol and Internet Protocol, TCP/IP), and/or physical interfaces may be used for the communication between HW and S-SW components.

Accordingly, in various examples, the marked physical HW components in FIG. 2A may be replaced by respective S-SW components. For example, one or more physical HW components may be replaced by specific dedicated computing devices, such as plug-in cards (or simulator cards) in a computer, or as standalone computing subsystems with their own dedicated physical interfaces to the medical imaging system. However, it is to be understood that simulation of the HW component may also be performed by a virtual computing device or cloud computing resource. The S-SW components may thus be cloud-implemented. At the same time, the communication interface HW component may be located on-site. Here, a conversion between a data bus communication protocol and Transmission Control Protocol / Internet Protocol may be implemented by the communication interface HW components.

The hybrid medical imaging system can include a housing such as a HW rack. One or more computing devices implementing the SW components and S-SW components can be arranged in the housing, as well as the HW components. This enables a modular exchange of a HW component by a respective S-SW component, or vice versa.

In various examples, the physical interfaces used between, i.e. to and/or from, one or more of HW components, SW components, or S-SW components, and/or any interface to the outside of the medical imaging system (i.e. peripheral devices) e.g. for receiving user input, receiving user output, providing data to display, receiving additional sensor data, data interfaces for transmitting measurement or imaging data to a backend or display devices, may be realized by any known HW interface. Such HW interfaces may be realized as wired or wireless interfaces, e.g., any known or dedicated physical computer port or application-specific wired electrical interface, using wireless communication protocols (e.g. a Bluetooth communication standard), optical interfaces (e.g. fiber optic connection) or any other current of future physical interface.

FIG. 2B schematically illustrates an exemplary hybrid medical imaging system 200 based on the CT imaging system of FIG. 2A, according to various examples.

As in FIG. 2A, the system architecture includes a HW subsystem (marked by dashed lines) and SW subsystem (marked by solid lines).

The hybrid medical imaging system 200 of FIG. 2B may also be referred to as partly simulated or hybrid digital twin of the CT imaging system of FIG. 2A, wherein, besides a number of S-SW components, also at least one real physical HW component is used and interfaced with the S-SW component(s).

As depicted in FIG. 2B, the software subsystem includes imaging control software component 210, an image reconstruction software component 209, and a control software component 208 for controlling simulated and physical HW components. These are native SW components. In the example of FIG. 2A, dedicated computing devices of the software components 208,209,210 are interfaced with each other via a protocol 216.

The hybrid medical imaging system 200 further includes a computing device 201 that implements multiple S-SW components. The S-SW components include a control software component 202. A plurality of S-SW components 203-207 are controlled by the control software component 202. The S-SW components 203-207 are each implemented by a respective dedicated computing device, specifically as a plug-in card in the computing device 201. In particular, each of the simulated HW components is realized as separate computing device with its own processor, memory, and its physical interfaces and communication protocols are the same as its original physical HW component, to which it corresponds and which it replaces. In various examples, SiDaNet, CANOpen, UART, CAN and similar interfaces and communication protocols may be used.

It is to be understood that the quantity of S-SW components is not limited to a specific number.

Each of the S-SW components 203-207 is interfaced via its specific physical interfaces and communication protocols with the control software component 208 and its dedicated computing HW, which implements the respective same counterpart physical HW interfaces as the simulated HW components. Therefore, the interactions between the S-SW components and the control software component 208, including the physical interfaces to the control software component 208, are the same as if the physical original HW components were used.

Furthermore, also one or more HW components 214 are present and used in the hybrid medical imaging system 200. Such physical HW components may be included as replacement for a S-SW component when the physical HW component becomes available during the development cycle. The HW component 214 is interfaced with control software component 208 via a specific physical interface and communication protocol.

The hybrid medical imaging system 200 further includes S-SW components 212, 213 that together provide a simulated execution of a detector function. This can be a simulation of a photon counting detector or other detector type. For instance, a simulation of photon counting or scintillation could be implemented. The S-SW component 212 simulates low level physical signal propagation and circuits of the detector, while the S-SW component 213 simulates (photon-)detector element interaction. Other detector variants may be simulated. The S-SW components 212, 213 are interfaced with the image reconstruction SW component 209 via communication protocol 216 and additionally with a data interface 215 for transmitting measurement data.

A user 211 interacts with and controls the simulator control SW component 202 and control SW component 210.

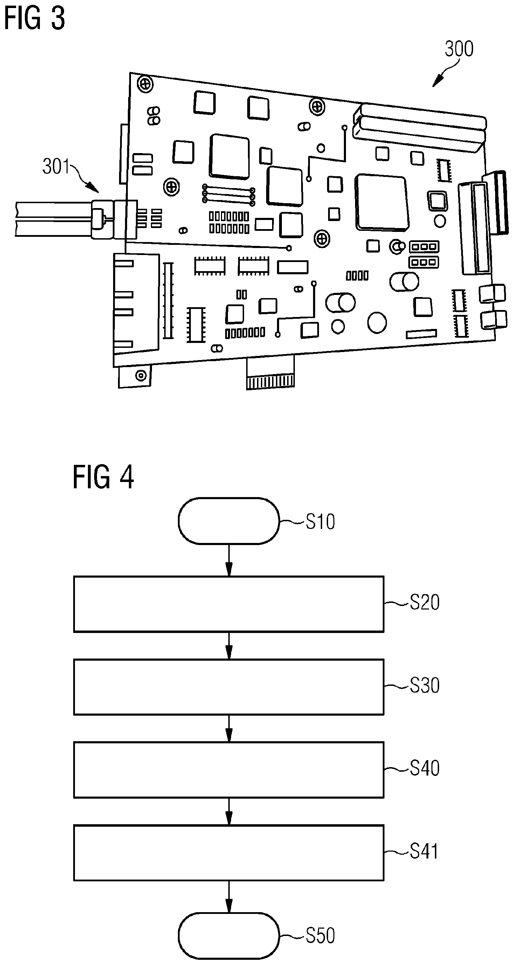

FIG. 3 schematically illustrates an exemplary computing device 300 implementing a S-SW component.

The computing device 300 of FIG. 3 is realized as plug-in card. The computing device 300 has a communication interface HW component 301. The communication interface HW component 301 emulates, towards other HW components (not shown in FIG. 3) the communication behavior. For example, the data bus system in a medical imaging system may run on a different voltage than a HW card such as the one illustrated in FIG.3. Therefore, for example, additional signal converters from 5 V to 24 V (or 12 V) or from 24 V (or 12 V) to 5 V may be used to provide the imaging system with the electrical signal it would expect from a physical HW component. For some fiber-optic interfaces specifically designed physical interfaces or plugs may be used, wherein no further signal converting may be necessary.

For simulating the behavior of the original physical components, e.g., a Field Programmable Gated Array (FPGA) chip may be used as computing device implementing the S-SW component. A firmware may be generated and copied on flash storage in the simulated HW component. The simulator firmware will be reloaded from flash storage to FPGA, if the system restarts and then communicate with the real imaging system. In such a way, the firmware stored in the simulated HW component may be updated to provide a different functionality, i.e. to simulate a different physical functions executed by HW components, and upon system restart, the simulated HW component will executed on the new firmware.

FIG. 4 schematically illustrates steps of a method, which can be executed for a simulated medical imaging system, according to various examples.

The method for a simulated medical imaging system starts in step S10.

In step S20, a first HW component is included in the HW subsystem, which provides execution of a first physical function.

In step S30, a S-SW component is included in the SW subsystem, which provides a simulated execution of a second physical function.

Examples for the second physical function include: a patient handling function, e.g., of a patient handling system onto which a patient can be placed and then moved towards a measurement position; a detector function, e.g., detecting waves or particles; a radiation source function, e.g., emitting waves or particles for measurement by the detector, e.g., an X-ray source function, an RF coil function, an ultrasound source function, etc.; a function related to moveable elements, e.g., a CT gantry function; a function related to magnetic elements, e.g., a DC magnet function or a gradient coil function; etc..

In step S40, the S-SW component included in step S30 is replaced with a further HW component, which provides execution of the second physical function (unsimulated).

Alternatively or additionally, the first HW component included in step S20 may be replaced by a further S-SW component, which provides simulation of the first physical function.

In step S41, the functionality of the hybrid medical imaging system can be tested. I.e., a validation of correct functioning of the individual components and/or the correct interoperability between pairs of the components can be tested. The testing can be based on monitoring physical functions that are executed or for which a simulated execution is provided. Testing the functionality of the hybrid medical imaging system can, in particular, include controlling the components - e.g., HW components and SW components and S-SW components - in accordance with an imaging protocol. The imaging protocol can pertain to a set of control commands that are predetermined and that facilitate to obtain medical imaging data, in the case of a hybrid medical imaging system simulated medical imaging data. Thereby, global functionality of the hybrid medical imaging system including interaction of the various components can be tested. Successful system integration can be tested.

The method ends in step S50.

Summarizing, simulated HW components (S-SW components) may be used in a hybrid medical imaging system, such as a CT system, as alternative to real HW components. This may be referred to as a partly simulated medical imaging system, or hybrid medical imaging system, or digital twin imaging system with at least one HW component. HW components may be modularly replaced by S-SW components, or vice versa. Thereby, shorter development and testing times may be realized.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

For illustration, above, various scenarios have been disclosed in connection with computed tomography (CT) imaging techniques. Similar techniques may be readily applied to various other kinds and types of digital imaging systems, for example medical imaging systems such as Magnetic Resonance Imaging (MRI, Microscopy, X-Ray, or Ultrasound Imaging (Sonography) systems, to name just a few examples.

## Claims

1. A hybrid medical imaging system, comprising:
- a hardware component configured to provide an execution of a first physical function of the hybrid medical imaging system; and
- a simulation software component configured to provide a simulated execution of a second physical function.

2. The hybrid medical imaging system of claim 1, further comprising:
- at least one computing device configured to simulate a physical interaction between the first physical function and the second physical function.

3. The hybrid medical imaging system of claim 2,
- wherein the physical interaction is selected from the group comprising a DC electric field, an AC electric field, a DC magnetic field, an AC magnetic field, vibration, a temperature gradient, and a pressure gradient.

4. The hybrid medical imaging system of any one of the preceding claims, further comprising:
- a communication interface hardware component configured to communicate with the hardware component and the simulation software component.

5. The hybrid medical imaging system of claim 4,
- wherein the communication interface hardware component is configured to communicate with the hardware component using a first communication protocol and to communicate with the simulation software component using a second communication protocol different than the first communication protocol.

6. The hybrid medical imaging system of any one of the preceding claims, further comprising:
- at least one computing device implementing the simulation software component, and
- a housing comprising the hardware component and the at least one computing device.

7. The hybrid medical imaging system of any one of the preceding claims,
- wherein the hybrid medical imaging system is a hybrid computed tomography medical imaging system,
- wherein the second physical function is an X-ray source function or an X-ray detector function.

8. The hybrid medical imaging system of any one of the preceding claims,
- wherein the second physical function is selected from the group comprising a patient handling function, a detector function, and a radiation source function.

9. The hybrid medical imaging system of any one of the preceding claims, further comprising:
- a software component configured to provide an execution of a software function.

10. The hybrid medical imaging system of any one of the preceding claims, further comprising:
- a control component configured to control the hardware component and the simulation software component based on an imaging protocol.

11. The hybrid medical imaging system of any one of the preceding claims,
- the simulated execution of the second physical function being dependent on the execution of the first physical function.

12. A method, comprising:
- including a hardware component in a hybrid medical imaging system, the hardware component being configured to provide an execution of a first physical function of the hybrid medical imaging system,
- including a simulation software component in the hybrid medical imaging system, the simulation software component being configured to provide a simulated execution of a second physical function of the hybrid medical imaging system,
- replacing the simulation software component by a further hardware component in the hybrid medical imaging system, the further hardware component being configured to provide an execution of the second physical function of the hybrid medical imaging system.

13. The method of claim 12, further comprising:
- replacing the hardware component by a further simulation software component in the hybrid medical imaging system, the further simulation software component being configured to provide a simulated execution of the first physical function of the hybrid medical imaging system.

14. The method of claim 12 or 13, further comprising:
- testing functionality of the hybrid medical imaging system based on monitoring the first physical function and the second physical function.

15. The method of any one of claims 12 to 14,
- wherein the hybrid medical imaging system is the hybrid medical imaging system of any one of claims 1 to 11.
